# EUROPEAN PATENT SPECIFICATION

(11) **EP 1 330 433 B1**
(45) Date of publication and mention of the grant of the patent: **24.01.2007**
(21) Application number: 01965401.1
(22) Date of filing: 04.09.2001
(51) Int. Cl.: C07C 305/10, C11D 1/16, C11D 1/29

(54) **SURFACTANT**
TENSID
TENSIOACTIF

(30) Priority: 04.09.2000 GB 0021633
(43) Date of publication of application: 30.07.2003
(73) Proprietor: Surfactant Technologies Limited, Earls Road Grangemouth Stirlingshire FK3 8XG (GB)
(72) Inventor: HARRISON, John, Edinburgh EH3 6LF (GB); ZWINDERMAN, Mark, Edinburgh EH3 9BN (GB)
(74) Representative: Gordon, Naoise Padhraic Edward
(86) International application number: PCT/GB2001/003953
(87) International publication number: WO 2002/020473

(56) References cited:
- EP-A- 0 832 965
- WO-A-98/00498
- FR-A- 2 733 982
- GB-A- 1 563 089
- US-A- 2 944 900
- US-A- 3 832 408
- PATENT ABSTRACTS OF JAPAN vol. 001, no. 013 (C-005), 22 March 1977 (1977-03-22) -& JP 51 128912 A (RIKEN VITAMIN CO LTD), 10 November 1976 (1976-11-10)
- PATENT ABSTRACTS OF JAPAN vol. 2000, no. 12, 3 January 2001 (2001-01-03) -& JP 2000 256549 A (KAO CORP), 19 September 2000 (2000-09-19)

## Description

This invention relates to surfactant compounds and to the use of the compounds in the formation of oil-in-water microemulsions.

Surfactants are surface active chemical agents. A surfactant molecule comprises a water soluble (hydrophilic) group and an oil soluble (hydrophobic) group. As such surface active molecules will align themselves at an oil/water interface with the water soluble group being solubilised into the water (aqueous) phase and the oil soluble group being solubilised into the oil (organic) phase accordingly.

The addition of surfactants to a mixture of oil and water either increases or decreases the extent to which the two liquids solubilise each other. Surfactants reduce the interfacial surface tension between the two immiscible liquids, enabling them to be dispersed within each other. Depending on the proportions and the precise nature of the chemical components either water-in-oil (W/O) or oil-in-water (O/W) dispersions may be produced. These mixtures are called emulsions. Most current commercially available surfactants, surfactant formulations and products used are emulsion formers or emulsion systems.

Emulsions have an inefficient molecular packing capability at the oil/water interface which in turn results in some direct oil/water contact at the interface. The uncoated surfaces at the interface are therefore directly exposed to the continuous phase. This is a thermodynamically unfavourable situation. As a result the droplets aggregate by coalescing at their exposed surfaces, increasing the surface area : volume ratio and hence minimising oil : water contact. Hence the outcome of extensive droplet coalescence is bulk-phase separation. Also as a result of the inefficient molecular packing at the interface emulsions have inherent higher surface tensions.

Emulsions are therefore turbid and may remain stable for a considerable length of time before phase separation occurs. Emulsions are multiple phase, cloudy colloidal systems in nature and, importantly, are likely to require an energy input in order to form.

Microemulsion systems, on the other hand, are defined as being thermodynamically stable (they form spontaneously on simple mixing at ambient temperatures and pressures). They are single-phase and optically transparent (isotropic) liquid mixtures of oil, water and amphiphile i.e. surfactant. As with emulsions microemulsions may be (O/W) or (W/O) systems.

In oil-in-water (O/W) microemulsion systems the continuous phase is water and the dispersed phase consists of a monodispersion of oil droplets, each coated with (and therefore encapsulated by) a close-packed monolayer of surfactant molecules. Water-in-oil (W/O) microemulsion systems are the inverse of this scenario where water is the dispersed phase and oil forms the continuous phase. These encapsulated droplet structures are referred to as micelles. In effect within microemulsions one phase is solubilised within the other.

Microemulsions are usually optically transparent, since the individual droplets are so small that they do not scatter visible light (having diameters in the region of only 3 - 200 nanometers). In comparison micelles in emulsion systems are typically larger than 200 nanometers and hence emulsions scatter visible light and appear turbid or opaque.

The inherent thermodynamic stability of microemulsion systems arises from the fact that, due to the close and efficient packing of the surfactant molecules at the monolayer interface, there is no direct oil/water contact. One result of this extremely efficent molecular packing is low or "ultra low" interfacial surface tensions which may be several orders of magnitude lower than those found in emulsion systems.

Apart from the significant physical differences described above, which can be determined by visual examination, emulsions and microemulsion systems can be distinguished further by measuring the surface tension at the oil-water interface. The surface tension at plain oil-water interfaces is typically of the order of 50 mNm⁻¹. Emulsions formed by mixing oil, water and an "ordinary" (i.e. emulsion-forming) surfactant are typically characterised by surface tensions in the region of 0.1 - 1 mM m⁻¹, whereas microemulsion systems are characterised by far lower surface tensions in the region of 10⁻³ - 10⁻⁶ mN m⁻¹. These latter values reflect the efficiency of the molecular packing at the oil-water interface and the complete absence of direct oil-water contact.

The differences in physico-chemical behaviour between emulsions and microemulsions described above are the characteristics that provide microemulsion systems with such unique and advantageous characteristics.

The prior art has demonstrated that certain chemicals eg. intermediate chain length alcohols can dissolve and solubilise important quantities of oil in water and it is thought that this is related to the microheterogeneity of the water/alcohol mixtures. The hydrocarbon fraction is preferentially soluble in the alcohol microphases which enables an increase in their formation. Many of these alcohols for example are efficient at solubilising light oils such as benzene or hexane in water.

However, prior research and development has shown that these systems are not efficient at dissolving heavier oils eg. decane, dodecane and tetradecane due to the insolubility of the alcohol in the heavier oils. Alcohols with longer chain lengths must be used for these types of oil but in turn they are not soluble in water. In order to modify these systems such that O/W microemulsions may be formed surfactants must be added to solubilise these alcohols into water. The relationship is synergistic as mutually the alcohols also increase the water solubility of the surfactants.

Prior art in the field has therefore shown that by combining surfactants or by combining surfactants with co-surfactants in the correct proportions oil dispersion capabilities in water are very significantly improved. In fact as a rule these combined "quaternary" surfactant/surfactant or surfactant/co-surfactant types of system are significantly more effective than the use of either surfactant or alcohol for example separately.

However, currently no effective ternary system adapted to form an O/W microemulsion is currently available commercially.

Many quaternary surfactant/surfactant or surfactant/co-surfactant systems are known in the prior art which are capable of forming both O/W and W/O microemulsions. The ability of surfactants to stabilise W/O microemulsions without the need for a co-surfactant is known in the art. For example sodium bis 2-ethylhexyl sulphosuccinate (Aerosol-OT), ammonium bis(ethylhexyl) hydrogen phosphate (HN₄DEHP), and didodecyltrimethyl ammonium bromide (DDAB) all preferentially form water-in-oil (W/O) microemulsions without the need for a co-surfactant or other chemical additive.

Many nonionic surfactants are known to be capable of forming such true ternary O/W microemulsion systems namely many of the Brij (Trade Mark) (polyoxyethylene ethers), Span (sorbitan esters), Tween (polyoxyethylene sorbitan esters), Myrj (Trade Mark) and other such families of surfactant.

However, there are many disadvantages of using microemulsions stabilised with known nonionic surfactants in many applications. For example known nonionic systems are known to be sensitive to very small changes in environmental variables such as temperature and salt concentration. As a result these systems form very "unstable" (single phase) microemulsions and exhibit very complex phase behaviours. Although cost efficient to manufacture microemulsions stabilised with known nonionic surfactants are very unpredictable and may thus be impractical to work with.

However, currently no effective ternary system adapted to form an O/W microemulsion is currently available commercially.

Accordingly, a need exists for "ternary" surfactant systems which preferentially are capable of forming oil-in-water (O/W) microemulsions without the need for co-surfactants and/or other chemicals. In particular, a need exists for anionic surfactants adapted to form O/W microemulsions.

This invention therefore relates to the design and synthesis of a range of specialist (oil-in-water) microemulsion forming surfactants for use (independently or as part of a chemical formulation) for any number of suitable industrial, environmental and domestic applications eg. the remediation of oil contaminated aquifers.

According to the invention there is provided the use of a compound in the formulation of an oil-in-water microemulsion, said compound having the general formula I 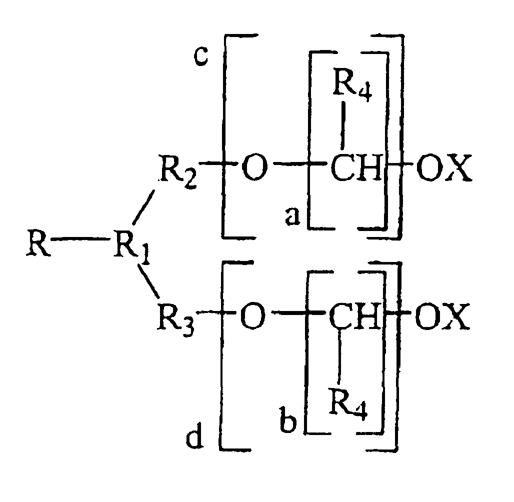 wherein R is a hydrocarbon group including H, branched or linear alkyl chains, substituted alkyl, alkenyl, aryl, alkaryl or cyclic groups;
R₁ is any of C, N, P, B, S, or SiO₄;
R₂ is any of a covalent bond, O, CH₂, (CH₂)ₙ where n may be 1 - 10 carbons long;
R₃ is any of a covalent bond, O, CH₂, (CH₂)ₙ where n may be 1 - 10 carbons long;
R₂ and R₃ may be the same or different;
R₄ is any of O, H, OH, CH₃, (CH₂)ₙCH₃, (CH₂)ₙOH, (CH₂)ₙOX or any combinations thereof where n is from 1 to 10.
OX is a water soluble group;
chains a and b are in the range 1 - 10 carbons each; and
the alkoxy chains c and d are in the range 1 - 20 units each.

In an alternative embodiment the invention also provides a compound having the general formula II 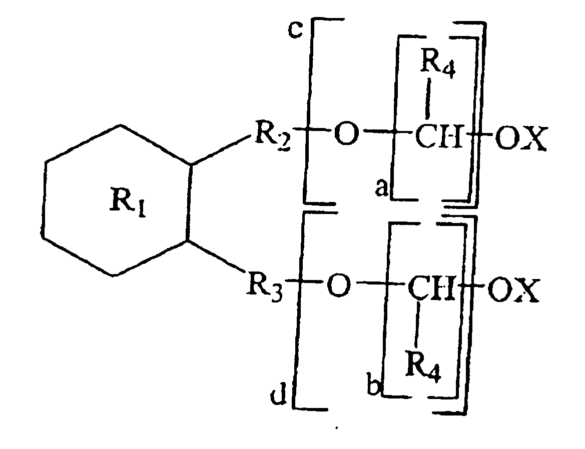 wherein R₁ is a cyclic hydrocarbon;
R₂ is any of a covalent bond, O, CH₂, (CH₂)ₙ where n may be 1 - 10 carbons long;
R₃ is any of a covalent bond, O, CH₂, (CH₂)ₙ where n may be 1 - 10 carbons long;
R₂ and R₃ may be the same or different;
R₄ is any of O, H, OH, CH₃, (CH₂)ₙCH₃, (CH₂)ₙOH, (CH₂)ₙOX where n is from 1 to 10;
OX is a water soluble group;
chains a and b are in the range 1 - 10 carbons each; and
the alkoxy chains c and d are in the range 1 - 20 units each.

The invention also extends to a compound having the general structure III 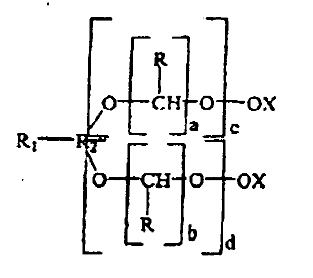 where R₁ is H, a branched or linear alkyl chain or a substituted alkyl, alkenyl, aryl, alkaryl or cyclic group;
R₂ is any of C, N, P, B, S, or SiO₄ or any subgroups thereof;
R is O, H, OH, CH₃, (CH₂)ₙCH₃, CH₂OH, (CH₂)ₙOX or any combinations thereof where n is from 1 to 10;
X is H, sulphate, sulphonate, carboxylate, a pH sensitive group, a mono- di- or oligo-saccharide or any combination thereof;
a and b in the range 1 - 10 each;
c and d are in the range 1 - 7 each.

In a further embodiment, the invention also extends to a compound having the general formula IV 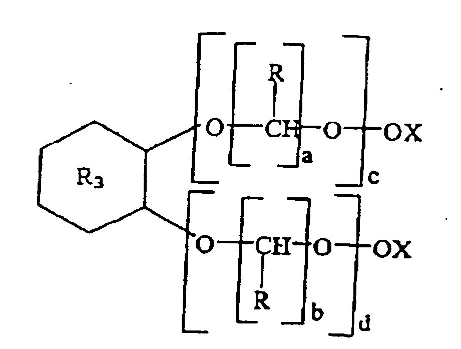 where R₃ is a cyclic hydrocarbon;
R is O, H, OH, CH₃, (CH₂)ₙCH₃, CH₂OH, (CH₂)ₙOX or any combinations thereof where n is from 1 to 10.
X is H, sulphate, sulphonate, carobxylate, a pH sensitive group, a mono- di- or oligo-saccharide or any combination thereof;
a and b are in the range 1 - 10.
c and d are in the range 1 - 7; and
the R₃ cyclic hydrocarbon is a C1 to C24 hydrocarbon.

The invention also extends to a surfactant comprising a compound as defined above, to a method of forming an oil-in-water microemulsion and to the use of a compound as defined above in the formation of an oil-in-water microemulsion.

The invention relates to the use of a compound in the formulation of an oil-in-water microemulsion, said compound having the general formula: 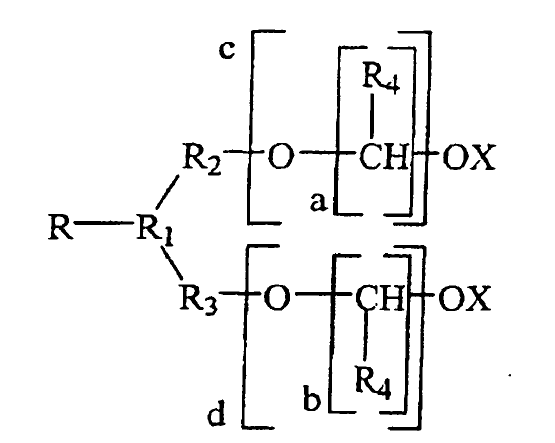 where R is a hydrocarbon group including H, branched or linear alkyl chains, substituted alkyl, alkenyl, aryl, alkaryl or cyclic groups. This may optionally include the non-hydrocarbon elements ethers or esters. The chain length of the group should be Cl to C30, preferably in the range C3 to C24, and most preferably C6 to C20;
R₁ is any of C, N, P,B, S, or SiO₄;
R₂ is any of a covalent bond, O, CH₂, (CH₂)ₙ where n may be of 1 - 10 carbons long and preferably 1 - 6 carbons long. The chain may be branched or unbranched;
R₃ is any of a covalent bond, O, CH₂, (CH₂)ₙ where n may be 1 - 10 carbons long and preferably 1 - 6 carbons long. The chain may be branched or unbranched;
R₂ and R₃ can be the same or different and may or may not be equal;
R₄ is any of O, H, OH, CH₃, (CH₂)ₙCH₃, (CH₂)ₙOH, (CH₂)ₙOX where n may be 1 - 10 and preferably is from 1 to 6 or any combinations thereof. R₄ groups on different chains can be the same or different and may or may not be equal;
OX is a water soluble group including but not limited to OH, sulphate, sulphonate, carboxylate, borate or a pH sensitive group eg. lactone, or any mono-di-or oligo-saccharide eg. galactose, fructose, sucrose, or maltose or any combination thereof. Di-anionic surfactants are preferred, most especially disulphate. The oxygen atom may or may not be present depending on the nature of the head group attached;
chains a and b are in the range 1 - 10 carbons each and are preferably 1 - 8 carbons and most preferably 1 - 4 ie. are alkoxy moieties preferably ethoxy, propoxy, butoxy or combinations thereof. The moieties may or may not be the same in each chain;
the alkoxy chains c and d are in the range 1 - 20 units each and are most preferably 2 - 10 units long. Chains c and d may differ in value;
non-hydrocarbon groups may or may not be included in the 2 to 5 head group chains. If the head group chains do not contain non-hydrocarbon groups then the chains are preferably at least 4 carbons in length. The same R₄ groups would apply as above.

In a second embodiment, the invention also extends to a compound having the general formula:

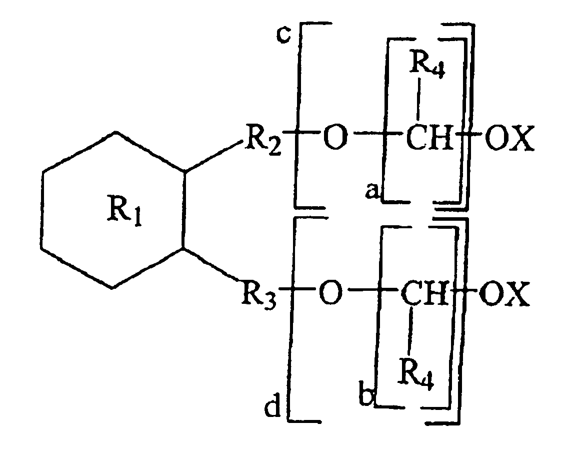

Where R₁ = a cyclic hydrocarbon. The cyclic hydrocarbon may be C1 to C24 in composition, preferably C4 to C20 and most preferably C4 - C12. The ring may or may not contain at least one double bond. The ring may also contain up to 4 other groups originating at any location. These adjoining groups may include branched or linear alkyl chains (branched or linear), substituted alkyl, alkenyl, aryl, alkaryl or further cyclic groups and any combinations thereof;
R₂ is any of a covalent bond, O, CH₂, (CH₂)ₙ where n may be 1 - 10 carbons long and preferably 1 - 6 carbons long. The chain may be branched or unbranched;
R₃ is any of a covalent bond, O, CH₂, (CH₂)ₙ where n may be of 1 - 10 carbons long and preferably 1 - 6 carbons long. The chain may be branched or unbranched;
R₂ and R₃ may or may not be equal;
R₄ is any of O, H, OH, CH₃, (CH₂)ₙCH₃, (CH₂)ₙOH, (CH₂)ₙOX where n may be 1 to 10 and is preferably 1 to 6 or any combinations thereof. R₄ groups on different chains can be the same or different and may or may not be equal;
OX is a water soluble group including but not limited to OH, sulphate, sulphonate, carboxylate, borate, pH sensitive group eg. lactone, or any mono-di- or oligo-saccharide eg. galactose, fructose, sucrose, or maltose or any combination thereof. Di-anionic surfactants are preferred, most especially disulphate. The oxygen atom may or may not be present depending on the nature of the head group attached;
chains a and b are in the range 1 - 10 carbons each and are preferably 1 - 8 carbons and most preferably 1 - 4 ie. are alkoxy moieties preferably ethoxy, propoxy, butoxy or combinations thereof. The moieties may or may not be the same in each chain;

The alkoxy chains c and d are in the range 1 - 20 units each and are most preferably 2 - 10 units long or any combinations thereof. Chains c and d may differ in value.

Non-hydrocarbon groups may or may not be included in the 2 to 5 head group chains. If the head group chains do not contain non-hydrocarbon groups then the chains are preferably at least 4 carbons in length. The same R₄ groups would apply as above.
Groups may originate from any position on the cyclic group but preferably 2 chains should originate from the 1,2 or 1,3 locations.

As described above the microemulsion fractions can potentially be treated to recover the oil-phase, preferably by adjusting the temperature, salinity, or pH. For ionic surfactants this results in, for example, a temperature induced phase separation, which yields an upper phase containing the oil and virtually no surfactant, and a lower phase of aqueous surfactant. The oil phase can be separated from the aqueous surfactant phase and both fractions can be recycled.

A microemulsion or microemulsion forming formulation including a surfactant of the invention is typically made up of a suitable aqueous or organic solvent.
Typically the solvent can comprise from 1 to 99% wt of the formulation. A suitable formulation may also include other surfactant (s) which may be non-ionic, anionic, cationic, zwiterionic, or amphoteric in nature and which may be used in appropriate proportions to enhance the capabilities, of the microemulsion or microemulsion forming system of the invention.

If desired a microemulsion or microemulsion forming surfactant formulation can comprise one or more co-surfactant(s) which may be used in appropriate proportions to enhance the capabilities of the microemulsion or microemulsion forming system.

One or more organic co-solvents may also be employed if desired in order to enhance the capabilities of the microemulsion or microemulsion forming system.

One or more chemical building agents may also be included in order to enhance the capabilities of the microemulsion or microemulsion forming system.
Similarly, chemical complexing or sequestering agents can be included in formulations in order to enhance the capabilities of the microemulsion or microemulsion forming system.

If desired, one or more chemical components which act as flocculating or coagulating agents can also be used in order to allow the flocculation of fines and hence prevent the build-up of fines in the formulated system.

The invention also extends to a compound having the general structure: 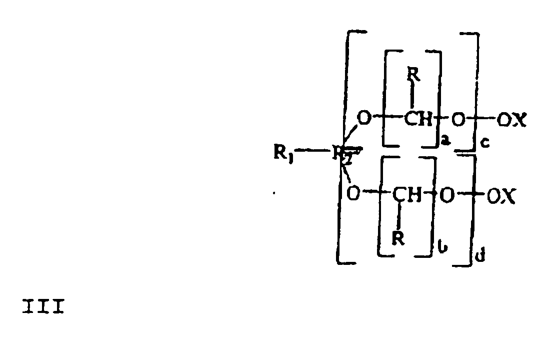 where R₁ is H, a branched or linear alkyl chain, or a substituted alkyl, alkenyl, aryl, alkaryl or cyclic group;
R₂ is any of C, N, P, B, S, or SiO₄ or any subgroups thereof and preferably carbon;
R is O, H, OH, CH₃, (CH₂)ₙCH₃, CH₂OH, (CH₂)ₙOX or any combinations thereof where n is from 1 to 10;
X is H, sulphate, sulphonate, carboxylate, a pH sensitive group e. g. lactone, or any mono- di- or oligio-saccharide e. g. galactose, fructose, sucrose, or maltose;
a and b are in the range 1 - 10 each and are preferably 1 - 8 carbons long and most preferably 1 - 4;
c and d are in the range 1 - 7 each and are preferably 1 - 5 units long or any combinations thereof;
R₁ may optionally include non-hydrocarbon elements e. g. ethers or esters.

The chain length of the group should be C1 to C28, preferably in the range C3 toC24, and most preferably C8 to C20.

Di-anionic surfactants are preferred, and more preferably is a disulphate.

Advantageously, a and b are in the range 1 to 4 such that they are alkoxy moieties preferably ethoxy, propoxy, butoxy or combinatins thereof. Most preferably the moieties are the same in each chain.
c and d may differ in value but preferably c and d are equal in number.

Non-hydrocrabon groups may or may not be included in the 2 to 5 head group chains. If the head chains do not contain non-hydrocarbon groups then the chains are preferably at least 4 carbons in length. The same R groups apply as above.

The invention also provides compounds having the general formula 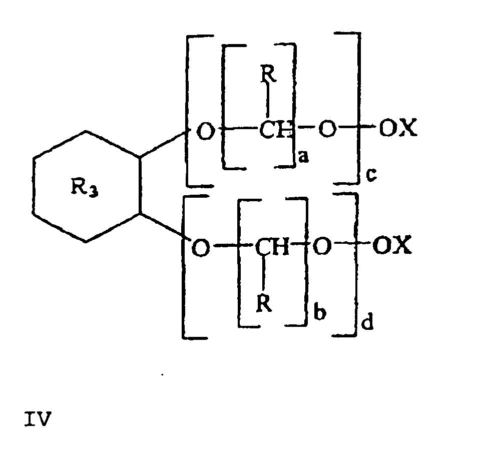 where R₃ is a C1 to C24 cyclic hydrocarbon which may or may not include non-hydrocarbon elements.
R is O, H, OH, CH₃, (CH₂)ₙCH₃, CH₂OH, (CH₂)ₙOX or any combinations thereof where n is from 1 to 10;
X is H, sulphate, sulphonate, carboxylate, a pH sensitive group e. g. lactone, or any mono- di- or oligio-saccharide e. g. galactose, fructose, sucrose, or maltose or any combination thereof;
a and b are in the range 1 - 10 each and are preferably 1 - 8 carbons long and most preferably 1 - 4;
c and d are in the range 1 - 7 each and are preferably 1 - 5 units long or any combinations thereof.

The R₃ cyclic hydrocarbon is preferably C4 to C20 and most preferably C6-C12. The ring may or may not contain any number of double bonds. The ring may also contain up to 4 other hydrocarbon groups or chains originating at any location. The ring may also contain adjoining R groups including alkyl groups (branched or linear) and further cyclic groups and any combinations thereof.

Di-anionic surfactants are preferred, most especially disulphate.
a and b are preferably in the range 1 to 4 to provide alkoxy moieties preferably ethoxy, propoxy, butoxy or combinations thereof. Most preferably the moieties are the same in each chain.
c and d may differ in value but preferably c and d are equal in number.

Non-hydrocarbon groups may or may not be included in the 2 to 5 head group chains. If the head chains do not contain non-hydrocarbon groups then the chains are preaferably at least 4 carbons in length.
Chains may originate from any position on the cyclic group but preferably 2 chains should originate from the 1,2 or 1,3 locations.

The invention therefore provides microemulsion forming surfactants developed for practical and cost efficient applications. These surfactants permit the use of O/W microemulsion and microemulsion forming systems in particular in suitable applications to replace more traditional emulsions and emulsion forming systems.

The surfactants of the invention are designed to be both easily biodegradeable in the environment and non-toxic in nature.

For example, in a preferred embodiment, the surfactants of the invention have just two head chains and as such the biodegradeability and thus the toxicity of the molecules is reduced as there is less branching in their structure. The compounds of the invention do not incorporate subgroups which may be released as toxic secondary breakdown products. This is a well known problem which has come to light with some of the more traditional surfactant types eg. those that contain benzene rings producing phenol groups as breakdown products (sodium dodecylbenzene sulphonate). Surfactant formulations employing the compounds of the invention therefore should not be dangerous, toxic, corrosive, flammable or explosive.

Because the compounds of the invention are microemulsion formers in their own right (forming true ternary systems) the previously necessary and costly development work and indeed use of complex surfactant formulations for many applications may be avoided. Accordingly, costs are reduced and environmental benefits are enhanced. The compounds of the invention may therefore be employed to produce simpler and more efficient surfactant based systems.

The advantages of the surfactant molecules and microemulsion systems containing the molecules are many depending on their application.

Because significant amounts of one phase may be efficiently solubilised in the other and because phase separation does not readily occur there may be aesthetic reasons for their application in many products.

Furthermore these molecules may be applied within extremely efficient cleaning systems across the board when compared to using emulsion forming surfactants and emulsion type formulation systems. This is due to both the inherent lower interfacial surface tensions achieved by microemulsions and the highly efficient solubilising capacity of microemulsions described above compared to emulsion systems.

Di-anionic surfactants are also known to have extremely strong detergency properties when compared to other types of surfactant eg. cationic surfactants. Using molecules which are known to have a very efficient level of detergency power has obvious advantages over other surfactants for general cleaning applications.

One advantage of the more advanced and specialised molecular designs of the invention is that they may be di- or even tri-anionic in nature (for example have twin sulphate or sulphonate head groups). Di-anionic surfactants are known to be less susceptible to some environmental variables such as changes in temperature or salt concentrations than are other types of surfactant - including some singularly anionic surfactants. Non-ionic surfactants in particular are known to be greatly affected by such variables. The same may be true for many quaternary systems - especially those which have a considerable nonionic constituent. Using the more robust surfactants of the invention may be highly advantageous when using these molecules in industrial or domestic applications where the environmental variables may be expected to change or be highly variable. On the whole di-anionic systems are therefore robust in nature.

Anionic surfactants are also known for their cost advantages in general as being some of the cheaper types of surfactant to manufacture on an industrial scale.

As indicated above, a considerable advantage of microemulsion systems is that the two different phases can be cleanly separated from each other. This is a very major advantage when considering industrial applications where emulsion formation is a problem causing difficult separation of the organic and aqueous phases. In addition normal emulsion forming surfactants have a tendency to contaminate the organic phase rendering the recycled organic product unusable for further applications.

Phase separation can be effected by several means depending on the design of the surfactant system. One method is by way of a thermally induced phase separation. For example, an O/W microemulsion may be warmed causing the surfactant to become more hydrophilic in nature (more soluble in the aqueous phase). The surfactant thus becomes preferably soluble in the aqueous phase and migrates into this phase from the micellar oil/water interface releasing the oil to the surface which phase separates out and rises to the surface as a less dense layer. Such mixtures may be separated using centrifugation techniques for example and the oil is tapped off leaving an aqueous surfactant (or a dilute O/W microemulsion containing only small amounts of oil depending on recycling efficiency) for recycling.

Alternatively if more concentrated surfactant solutions are formed and utilised at higher temperatures the O/W microemulsion may be cooled recrystallising the surfactant as the temperature drops below the Critical Micellar Temperature (CMT) which again releases the oil to the surface.

In addition if the surfactant is designed such that the molecules contain a pH sensitive group eg. an amine or a lactone group the surfactant may be precipitated out of solution or the molecular solubility characteristics changed by adjusting the pH accordingly, via addition of chemicals or the application of an electrical current, changing the physico-chemical behaviour of the surfactant and releasing the oil to the surface for separation. On readjusting the pH the surfactant may be converted back into its original form releasing it back into a solution for recycling.

Another technique that may be used is to adjust the salt concentration which, under suitable circumstances for certain surfactants, inflicts a change in the Winsor type system which can result in the release of oil. Ultra-filtration, ultracentrifugation, and coalescing techniques/chemicals may all be utilised to facilitate the recovery of non-polar substances from the O/W microemulsion.

Yet another technique is to precipitate the surfactant out of solution by chemical reaction which would allow phase separation to occur releasing surfactant free oil. This step may or may not be reversible depending on the chemistry involved.

Indeed phase separation using microemulsion systems may not even need to be inflicted depending on the type of microemulsion system utilised. For example if a Winsor I type system is used (an O/W microemulsion in the presence of an excess oil phase) a significant amount of the oil is naturally recycled to the surface of the system. Furthermore the oil recovered again remains essentially surfactant free and may be applied in turn to further uses.

Another advantage in increasing the anionic nature of these surfactants for environmental uses is that they naturally repel fine colloidal clay particulates which have a like negative charge. Fines and colloidal clays are a common problem with aqueous systems and using this type of molecule ensures that the fines is separate out more easily and less surfactant lost to the environment adhering to the surface of the solids.

Readily available flocculating agents may also be utilised effectively within water continuous O/W microemulsion systems. This has not been the case when emulsion systems are used as oil contact with the flocculating / coagulating agents has occurred disrupting the system and the action of the chemical agents added. (Since O/W microemulsions are water continuous and there is no direct contact with the oil no interference occurs).

Various embodiments of the invention will now be described, by way of example only, having regard to the accompanying drawings in which:
Figure 1 is a pseudoternary phase diagram of (SDS + B ratio, 1:1 by weight) in 0.58M NaCl and Novatec (Trade Mark) Base Fluid at 25° Celsius;
Figure 2 is a ternary phase diagram of Nonionic Union Carbide surfactant Triton RW-50(Trade Mark) in 0.58M NaCl and Novatec B (Trade Mark) Base Fluid at 25°C.
Figure 3 is a ternary phase diagram of AOT (Trade Mark) in 0.025M NaCl and Heptane at 25°C.
Figure 4 is a quarternary phase diagram of AOT (Trade Mark) (at 1:2 weight ratio), water and Novatec (Trade Mark) Base Fluid at 25°C).
Figure 5 is a shows molecular structure of known W/O microemulsion forming surfactants - AOT (Trade Mark) and NH₄DEHP respectively.
Figure 6 is a schematic representation of the theory behind the surfactant / co-surfactant monolayer structure in the SDS/B O/W microemulsion system.
Figure 7 is a schematic representation of the first step to O/W microemulsion forming surfactant design;
Figure 8 is a schematic representation of the second step to O/W microemulsion forming surfactant design;
Figure 9 is a schematic representation of the final step in O/W microemulsion forming surfactant design;

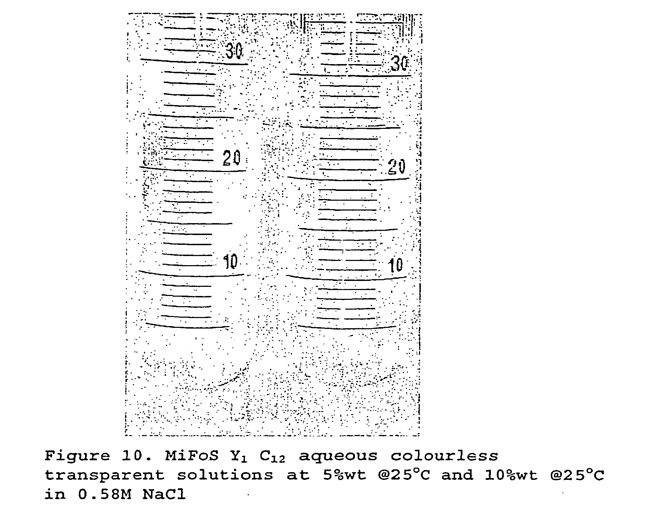

Figure 10 is a photograph of MiFoS Y₁ C₁₂ aqueous colourless transparent solutions at 5%wt @25°C and 10%wt @25°C in 0.58M NaCl
Figure 11 is a photograph of a crude oil contaminated sample treated with 10%wt MiFoS Y₁ C₁₂ in 0.58M NaCl @25°C in which formation of the oil-in-water (O/W) microemulsion and complete removal of surfactant free oil is apparent;
Figure 12 is a synthesis pathway for the sulphated Triton RW-50 reaction (non-ionic to di-anionic tertiary amine oil-in-water O/W microemulsion forming surfactant)(Y-shaped surfactant);
Figure 13 is a synthesis pathway for manufacturing an O/W microemulsion forming surfactant (V-shaped surfactant); and

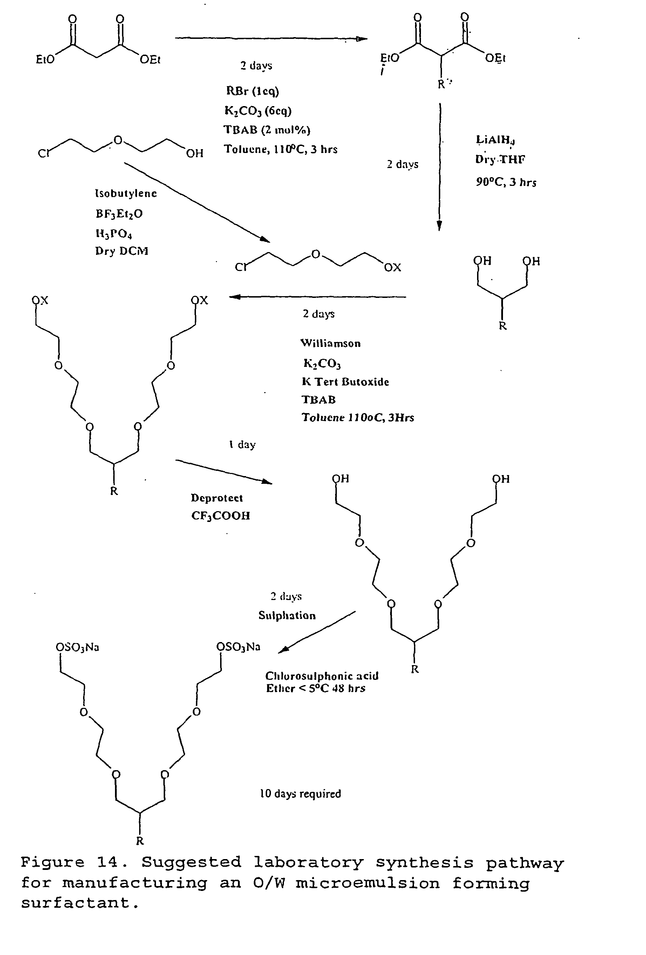

Figure 14 is an alternative synthesis pathway for manufacturing an alternative O/W microemulsion forming surfactant.

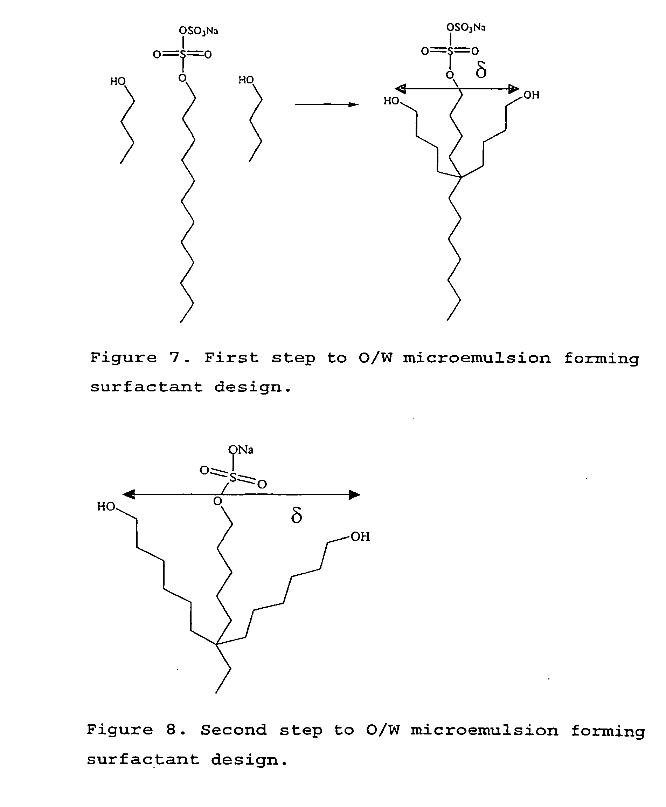

It has been shown that chemicals such as alcohols have the potential to produce strong hydrogen bonds with water. The alcohol co-surfactants also have a significant effect in altering the Hydrophile-Lipophile-Balance (HLB) of the aqueous system which must be compatible with the required HLB of the oil to be solubilised if oil solubilisation and microemulsion formation is to occur. In addition it has been shown that quarternary systems have a large degree of flexibility in the molecular composition of the interface. This allows for a fluid and continuous change in the proportions of each constituent as shown in Figure 8. The result is an extremely adaptive system capable of instantaneous alteration at the interface as required.

The surfactant components of the invention have structural properties which stabilise O/W microemulsions without the need for a co-surfactant or other chemical additives.

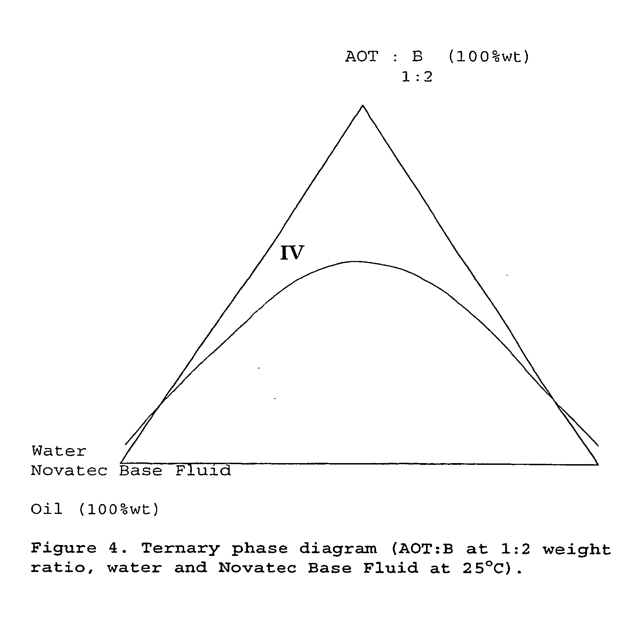

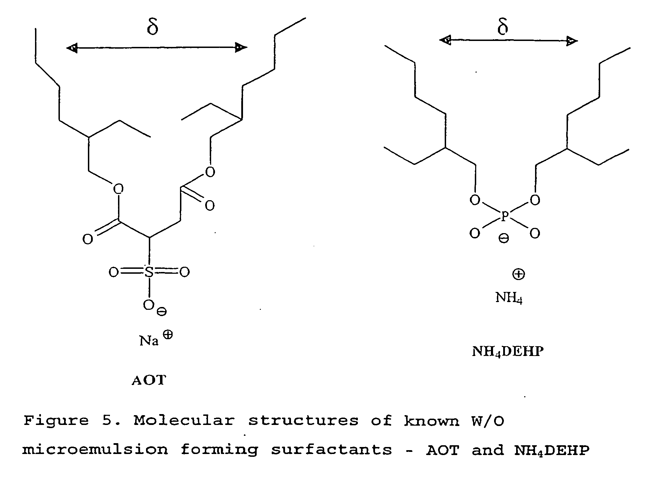

As stated above anionic water-in-oil (W/O) microemulsion forming surfactants such as Aerosol-OT (Trade Mark) (AOT) for example and other surfactants eg. ammonium bis(ethylhexyl) hydrogen phosphate (NH₄DEHP) are well known and some are readily available at industrial scales. The structures of some of these types of molecule are shown in Figure 5. They are described as true ternary systems as they do not require the use of other surfactants or co-surfactants in order to form W/O microemulsion systems. Their W/O microemulsion forming properties are well understood and are well documented in the literature. In turn these surfactants may be combined with co-surfactant chemicals to enhance their microemulsion forming capabilities and improve the flexibility of the systems such that they may also form quaternary O/W W/O microemulsions (for example see the quaternary phase diagram in Figure 4 for AOT).

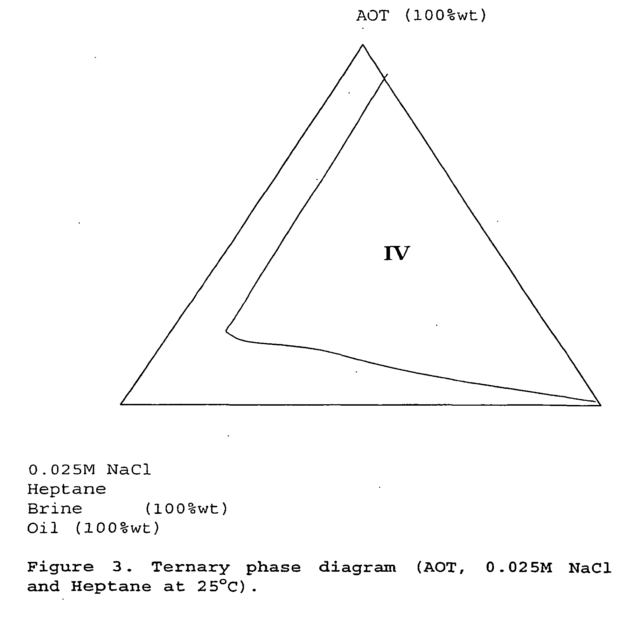

Surfactant molecules of this type are able to form very large single phase (Winsor IV) regions within their ternary phase diagrams (see AOT ternary phase diagram Figure 3). In' many cases very significant amounts of water can be taken up into an oil continuous W/O microemulsion using relatively small quantities of surfactant.

Although the applicants do not wish to be bound by any particular theorem it is thought that much of the above capabilities are due to the fact that the molecules are able to form a cone or V-shaped structure at the oil-water interface. The molecules possess a large degree of inherent flexibility in their structure indicated by δ in Figure 5. In this fashion the shape of the cone structure of the molecules is highly variable. This capability also allows the close packing efficiency of the molecules at the interface. These characteristics provide these systems with similar capabilities to quaternary systems described above.

As a result of adjusting the angles of the cone (unit shape) to effect the radius of curvature of the interface the size and shape of the micelles formed can vary accordingly depending on variables such as the quantities of water taken up into the system, the surfactant concentration, and other environmental variables eg. the salt concentration and temperature.

The Applicants have succeeded in replicating the above phenomenon in O/W forming surfactants.

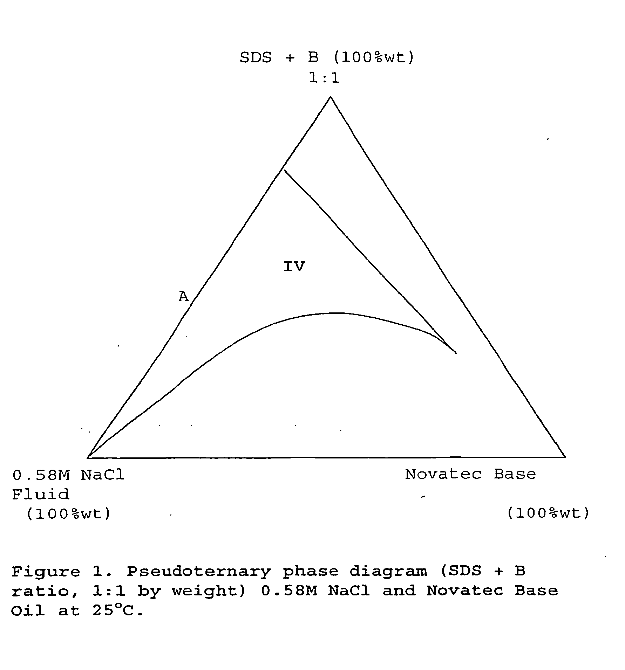

Surfactant behaviour can be quantitated in terms of a triangular phase diagram. The phase diagram for the quaternary O/W microemulsion system water / (SDS+B) / oil is shown in Figure 1. Here, (SDS+B) is a mixture of the surfactant sodium dodecyl sulphate (SDS) and butan-1-ol (B). B acts as a co-surfactant in the system, enhancing the O/W microemulsion-forming properties of SDS. As long as the SDS and B are held at constant ratio, they can be treated as a single component for the purpose of constructing the phase diagram.

The phase diagram in Figure 1 was constructed with the SDS : B ratio held at 1 : 1 by weight at 25°C. The oil used was a medium chain length oil (C14 - C16) Novatec (Trade Mark) B Linear Alkyl Olefin (LAO) Base Fluid supplied by M-I Drilling Fluids UK Ltd. This oil was a typical base fluid used in the preparation of oil based drilling muds for industrial use in the oil and gas industry.

The apexes of the phase triangle each correspond to one of the components in 100% pure form by weight ie. oil, water, or (SDS+B) at the stated ratio. Any point on one of the vertices between two of these points corresponds to a mixture of those two components in a defined ratio (given in percent weight - %wt). Thus point A on the water-surfactant axis in Figure 1 corresponds to a system containing (SDS + B) and water in 40:60 %wt ratio respectively. Any point within the triangle corresponds to a unique combination of the three components in a defined ratio. The physical state of the mixture at equilibrium is mapped onto the phase diagram. The phase triangle in Figure 1 is characterised by the prominent single phase microemulsion region, known as a Winsor IV system, which extends from the SDS+B/water axis towards the SDS+B/oil axis.

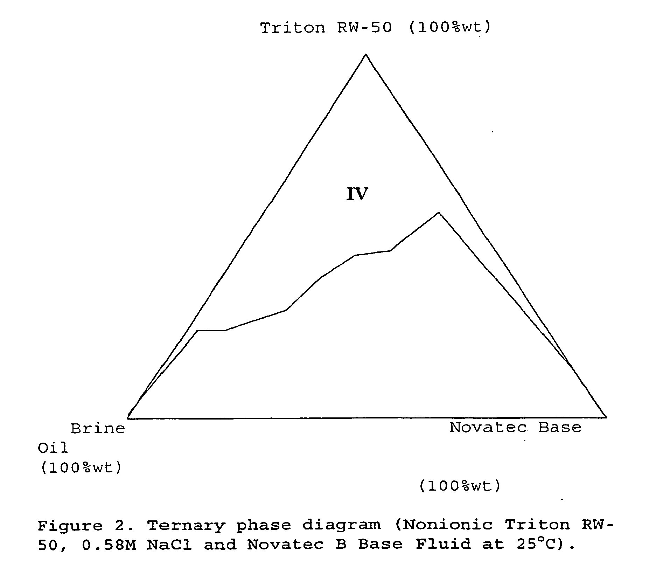

(A similar phase diagram in Figure 2 shows the Winsor IV region obtained for a known true ternary nonionic microemulsion forming surfactant manufactured by Union Carbide - Triton RW-50 (Trade Mark).

An O/W microemulsion was therefore developed in using the emulsion forming surfactant sodium dodecyl sulphate (SDS). SDS requires a co-surfactant butanol (B) to be added to the system in order that O/W microemulsions can be formed with medium chain length oils. As a result, the system used was termed a pseudoternary or quaternary since the surface active agent at the oil-water interface comprised two separate constituents.

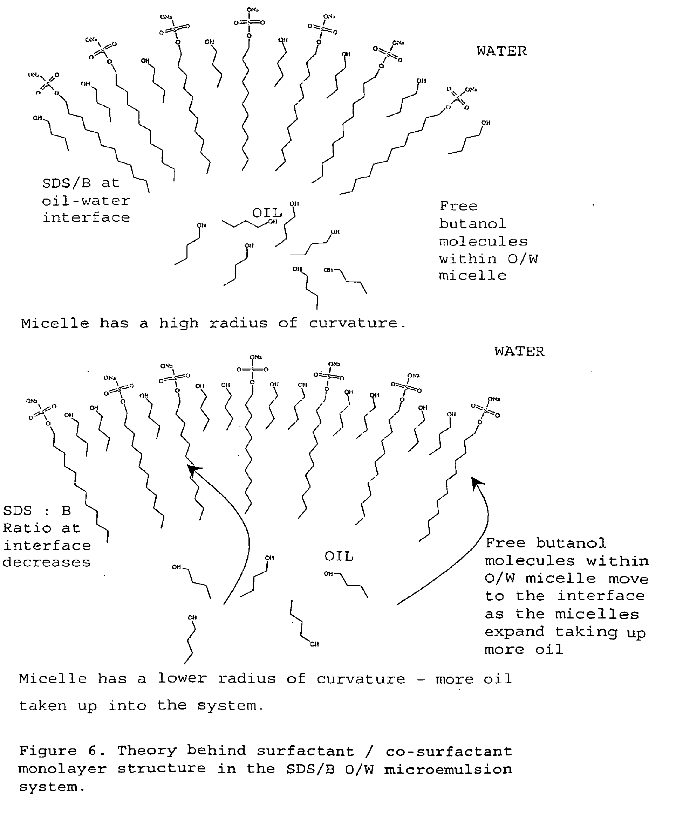

When using the SDS/B prototype system an excess of butanol is added. Butanol is only partially soluble in aqueous media (91mlL⁻¹ H₂O at 25°C) but is completely miscible with ether and organic solvents. The majority of the butanol in the microemulsion prototype system therefore resides at the oil-water interface of the micelles or within the oil phase within the micellar structures themselves. As more oil is taken up into the microemulsion system some of the excess butanol within the micelles may migrate to the interface in order to allow the micelles to expand. The effect of this migration is to increase the ratio of SDS:B at the interface and thus to change the angle of the cone formed by the SDS/B unit structures. This process allows more oil to be taken up into the system and is shown diagrammatically in Figure 6.

The SDS/B system was mimicked by combining the co-surfactant and the surfactant molecules together in a suitable ratio into one molecule in its own right such that the above characteristics of the SDS/B system could be duplicated in one molecular unit at the interface. The most appropriate method of achieving this was to attach the butanol molecules at their base to the SDS molecule in such a fashion that the hydroxyl groups were maintained at the interface alongside the sulphate head group whilst still maintaining the molecular inherent flexibility such that it may adjust the angle of the unit cone formed in the same way as in the SDS/B system. The result was a molecule as shown in Figure 7 which is generally Y shaped in structure.

This molecule was developed still further to increase the inherent flexibility in the molecule to form a more V shaped molecule as shown in Figure 8. In all cases a similar number of hydrocarbon (water insoluble / hydrophobic) and non-hydrocarbon (water soluble / hydrophilic) groups of the molecule were maintained in order to keep the same Hydrophile - Lipophile Balance (HLB) of the system.

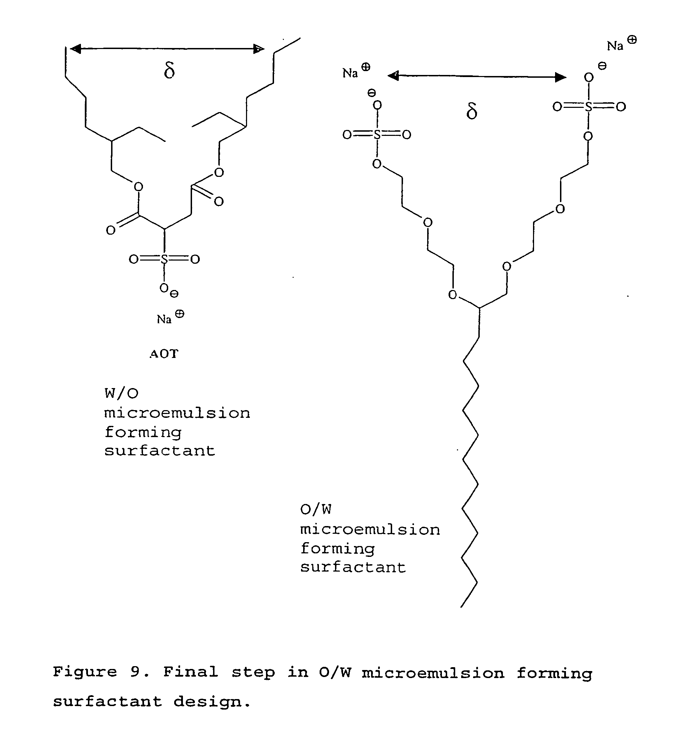

The Y and V shaped molecules were further modified to more closely resemble a mirror image of AOT type molecules i.e. instead of having a water soluble tail group and oil soluble head groups the molecule was designed to have an oil soluble tail group and water soluble head groups as shown in Figure 9.

The ratios of hydrocarbon (water insoluble / hydrophobic) and non hydrocarbon (water soluble / hydrophilic) groups of the molecule may be adjusted in order to change the Hydrophile - Lipophile Balance (HLB) of the molecules depending on the required HLB of the oil to be solubilised into the O/W microemulsion.

The microemulsion forming surfactants outlined herein are preferably anionic or nonionic Y (and V shaped) surfactant molecules with microemulsion forming capabilities whose generic designs are outlined by the parameters laid out below. Anionic molecules may have an alkali or alkaline earth metal counter-ion eg. Na, Mg, Ca, K, or a substituted or un-substituted ammonium ion etc.

This invention will now also be described having regard to the following non-limiting examples:

### Examples

In the examples the amounts of oil taken up into di-anionic O/W microemulsion systems were studied using a cloud point titration method. A pure oil was slowly titrated into a weighed amount of the transparent aqueous surfactant solution comprising a known surfactant concentration by weight. The systems were left to equilibrate overnight after each addition of oil. The titration with oil was continued until the surfactant solution reached the cloud point and the system ceased to solubilise the oil and thus became opaque. From this point phase separation occurred releasing the excess unsolubilised pure oil to the surface (Winsor I system). The percentage weight (%wt) of oil that can be solubilised into each transparent and stable aqueous O/W microemulsion (ie. does not phase separate) could then be calculated.

All studies were carried out at ambient temperature (25°C) and at atmospheric pressure.

### Example 1

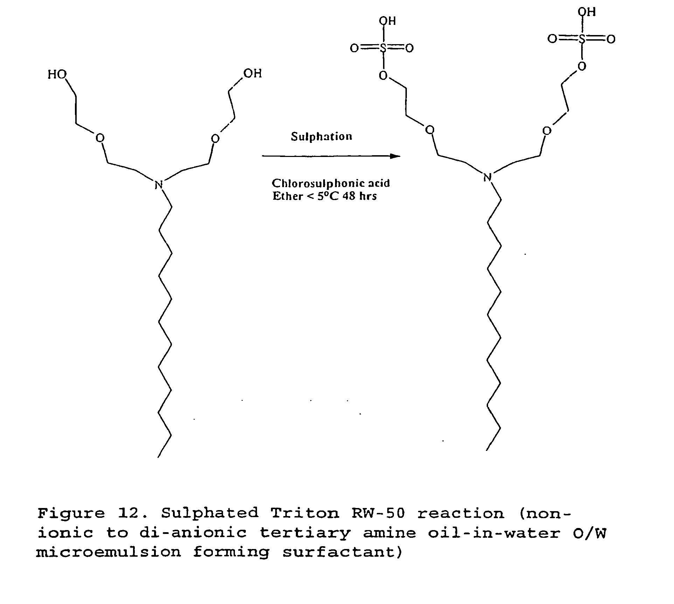

The nonionic Union Carbide amine based surfactant (Triton RW-50) (Trade Mark) was sulphonated using known industrial chemical methods in the laboratory shown in Figure 12. This produced a di-anionic surfactant product which was found to be readily soluble in distilled water at neutral pH. 100g of a 50%wt surfactant solution in distilled water was prepared. This was titrated with toluene (SD=0.865) until the cloud point was reached. The system took up 30mls of toluene into an optically transparent O/W microemulsion. This equated to 25.95g and a system containing 20.6%wt oil before the aqueous O/W microemulsion system became opaque.

### Example 2

As in Example 1, 100g of a 30%wt aqueous surfactant solution using the above di-anionic surfactant product was prepared in distilled water. This system was titrated with heptane (SD=0.684). The system took up 62.5mls of heptane into a transparent O/W microemulsion before the cloud point was reached. This equated to 42.8g or 29.94%wt oil solubilised.

### Example 3

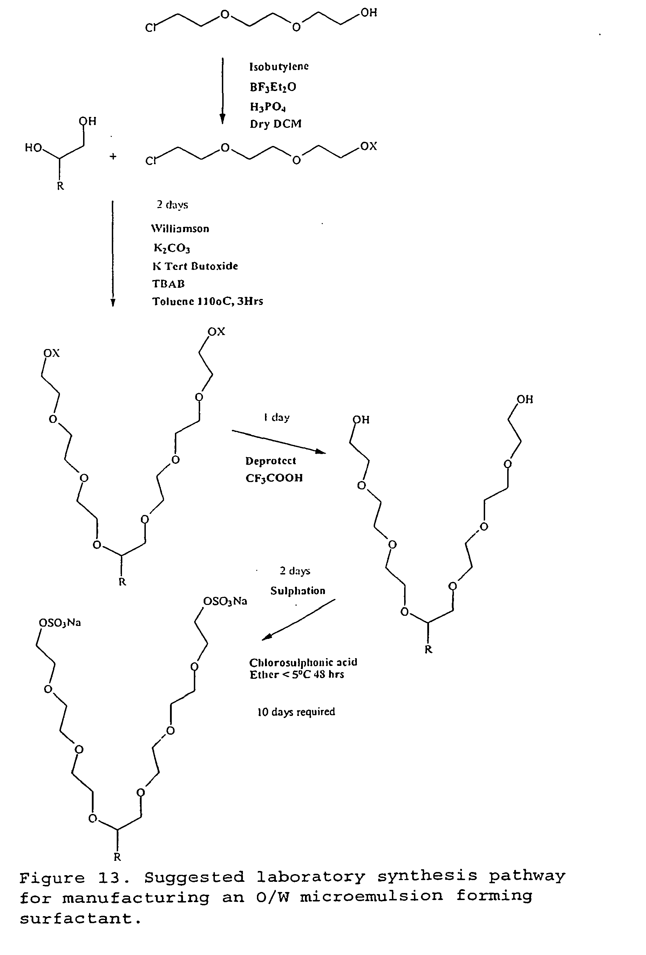

A synthesised di-anionic carbon based ethoxylated surfactant as outlined in Figure 13 with a carbon tail chain length of C₁₂ was employed. The surfactant was readily soluble in water at low temperatures. A 40%wt aqueous surfactant solution was prepared using an OECD standard sea water (brine) which contained 34g +/- 0.5g NaCl L⁻¹ in water (0.58M NaCl). This surfactant system was titrated with heptane (SD=0.684). The system took up 81.6mls heptane before the cloud point was reached. This was equivalent to 55.84g or 35.84%wt oil.

### Example 4

A synthesised carbon based ethoxylated di-anionic surfactant as outlined in Figure 13 with a carbon tail chain length of C₁₄ was employed. Again the surfactant was readily soluble in water at temperatures below 60°C. A 30%wt aqueous surfactant solution was prepared again using an OECD standard sea water (brine) containing 34g +/- 0.5g NaCl L⁻¹ in water (0.58M NaCl). This surfactant system was titrated with a medium chain length (C₁₄-C₁₆) Linear Alkyl Olefin (LAO) synthetic base oil (Novatec Base Fluid) (Trade Mark) supplied by MI Drilling Fluids UK Ltd (SD=0.771). The system was capable of taking up 10mls of this oil into an O/W microemulsion before the cloud point was reached. This was equivalent to 7.71g or 7.16%wt oil.

### Example 5

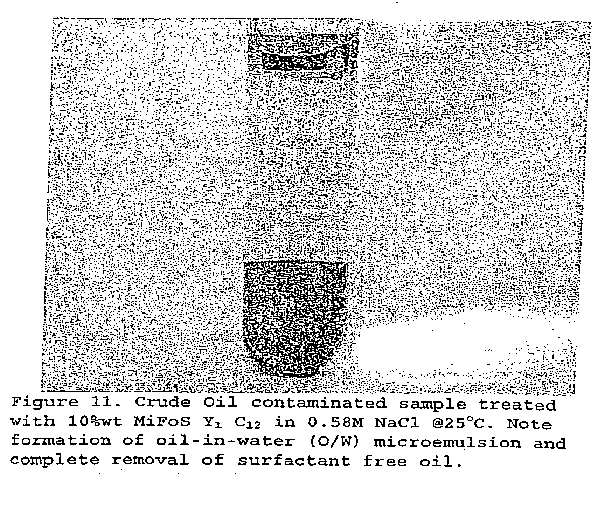

Figures 11 and 12 should be referred to. In this example a long chain oil contaminated sample has been shaken for a period of 2 minutes with a 10%wt surfactant solution in brine as was used and demonstrated in Example 3 above. It can be clearly seen that a Winsor I system was formed. The contaminated sample has been thoroughly cleaned of the crude oil which is released to the surface as a pure oil phase (free of surfactant) and a transparent O/W microemulsion has been formed in the aqueous surfactant phase.

Surfactants of the invention are therefore capable of forming true ternary O/W microemulsion systems. This has been demonstrated when using both distilled water and brine as the aqueous phase. In addition it has been demonstrated that both light oils and heavier oils can be solubilised into O/W microemulsion systems using these molecular designs. Furthermore the cleaning capabilities of these surfactant systems and the recovery of surfactant free oil from the contaminated sample has been demonstrated. In all cases these results have been achieved without the need for mixing or formulating surfactants and indeed the requirement of co-surfactant and/or co-solvent chemical additives has not been necessary. These are thus true ternary O/W microemulsion systems using di-anionic microemulsion forming surfactants.

Accordingly, the invention provides novel and inventive molecules with surface active properties providing the surfactant molecules with suitable microemulsion forming capabilities. These microemulsion forming surfactants have been facilitated for the practical and cost efficient use of the technology for a variety of industrial, environmental and domestic applications. The designs outlined herein may enable and permit the use of, in particular, di-anionic O/W microemulsion (forming) surfactants and microemulsion surfactant based formulations in such applications to replace more traditional emulsion forming surfactants, emulsion forming surfactant formulations and emulsion systems. Examples of applications of this technology and product formulations therefore include, but are not limited, to the remediation of oils from (ground) water and aquifers.

However, if desired, the molecular designs outlined herein may be combined with other chemicals in suitable proportions in order to increase the microemulsion capabilities of these systems.

## Claims

1. Use of a compound in the formulation of an oil-in-water microemulsion, said compound having the general formula I 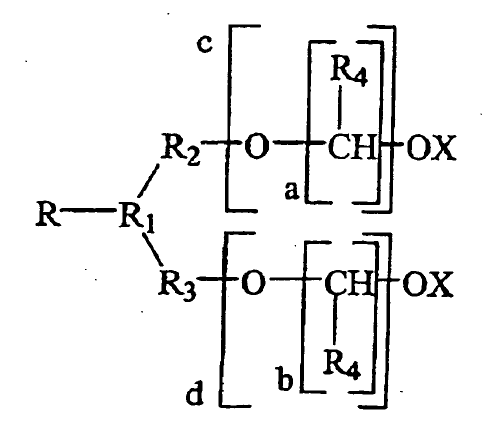 wherein R is a hydrocarbon group including H, branched or linear alkyl chains, substituted alkyl, alkenyl, aryl, alkaryl or cyclic groups;
R₁ is any of C, N, P, B, S, or SiO₄;
R₂ is any of a covalent bond, O, CH₂, (CH₂)ₙ wherein n may be 1-10 carbons long;
R₃ is any of a covalent bond, 0, CH₂, (_{CH2})ₙ where n may be 1-10 carbons long;
R₂ and R₃ may be the same or different;
R4 is any of O, H, OH, CH₃, (CH₂)ₙCH₃, (CH₂)ₙOH, (CH₂)ₙOX or any combinations thereof where n is from 1 to 10.
ox is a water soluble group;
chains a and b are in the range 1-10 carbons each; and
the alkoxy chains c and d are in the range 1-20 units each.

2. The use as claimed in Claim 1 wherein R further comprises an ether group or an ester group.

3. The use a compound as claimed in any one of Claims 1 or 2 wherein R has a chain length of C1 to C30.

4. The use as claimed in any one of Claims 1 to 3 wherein R has a chain length of C3 to C24.

5. The use as claimed in any one of Claims 1 to 4 wherein R has a chain length of C6 to C20.

6. The use as claimed in any one of Claims 1 to 5 wherein n in the (CH₂)ₙ group of R₂ is from 1 to 6.

7. The use as claimed in any one of Claims 1 to 6 wherein n in the (CH₂)ₙ group of R₃ is from 1 to 6.

8. The use as claimed in any of Claims 1 to 7 wherein OX is selected from the group comprising OH, sulphate, sulphonate, carboxylate, borate, or a pH sensitive group.

9. The use as claimed in Claim 8 wherein the pH sensitive group is selected from the group comprising lactone, or a mono-, di- or oligo-saccharide.

10. The use as claimed in Claim 9 wherein the saccharide comprises galactose, fructose, sucrose, or maltose or any combination thereof.

11. The use as claimed in any of Claims 1 to 10 wherein a and b range from 1 to 8 carbons.

12. The use as claimed in any of Claims 1 to 11 wherein a and b range from 1 to 4.

13. The use as claimed in any of Claims 1 to 12 wherein the alkoxy chains c and d comprise from 2 to 10 units.

14. A compound having the general formula II 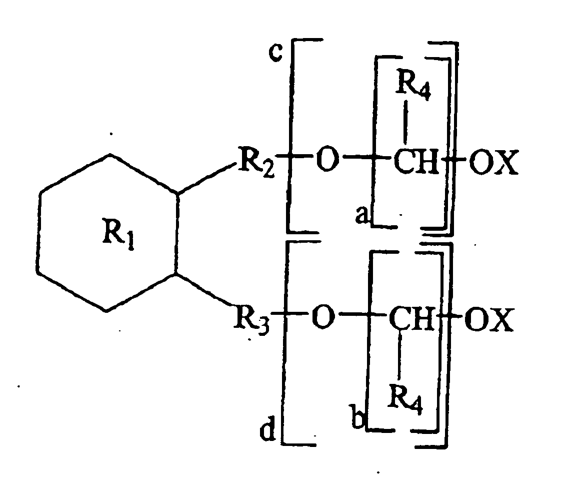 wherein R₁ is cyclic hydrocarbon;
R₂ is any of a covalent bond, O, CH₂, (CH₂)ₙ where n may be 1-10 carbons long;
R₃ is any of a covalent bond, O, CH₂, (CH₂)ₙ where n may be 1-10 carbons long;
R₂ and R₃ may be the same or different; R₄ is any of O, H, OH, CH₃, (CH₂)ₙCH₃, (CH₂)ₙOH, (CH₂)ₙOX where n is from 1 to 10;
OX is a water soluble group;
chains a and b are in the range 1-10 carbons each; and
the alkoxy chains c and d are in the range 1-20 units each.

15. A compound as claimed in Claim 14 wherein the cyclic hydrocarbon is a C1 to C24 cyclic hydrocarbon.

16. A compound as claimed in Claim 14 or Claim 15 wherein the cyclic hydrocarbon is a C4 to C20 hydrocarbon.

17. A compound as claimed in any of Claims 14 to 16 wherein the cyclic hydrocarbon is a C4 to C12 hydrocarbon.

18. A compound as claimed in any of Claims 14 to 17 wherein the cyclic ring comprises at least one double bond.

19. A compound as claimed in any of Claims 14 to 18 wherein n in the (CH₂)ₙ group of R2 is from 1 to 6.

20. A compound as claimed in any of Claims 14 to 19 wherein OX is selected from the group comprising OH, sulphate, sulphonate, carboxylate, borate, or a pH sensitive group.

21. A compound as claimed in Claim 20 wherein the pH sensitive group is selected from the group comprising lactone, or a mono-, di- or oligio-saccharide.

22. A compound as claimed in Claim 21, wherein the saccharide comprises galactose, fructose, sucrose or maltose or any combination thereof.

23. A compound as claimed in any of Claims 14 to 22 wherein a and b range from 1 to 8 carbons.

24. A compound as claimed in any of Claims 14 to 23 wherein a and b range from 1 to 4.

25. A compound as claimed in any of Claims 14 to 24 wherein the alkoxy chains c and d comprise from 2 to 10 units.

26. A compound having the general structure III 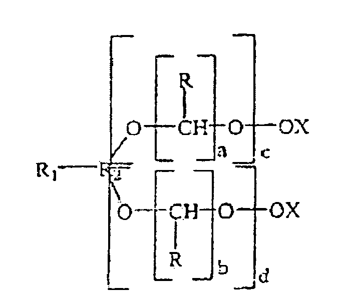 where R₁ is H, a branched or linear alkyl chains, or a substituted alkyl, alkenyl, aryl, alkaryl or cyclic groups;
R₂ is any of C, N, P, B, S, or SiO₄ or any subgroups thereof;
R is O, H, OH, CH3, (CH2)nCH3, CH2OH, (CH2)nOX or any combinations thereof where n is from 1 to 10;

27. A compound having the general formula IV 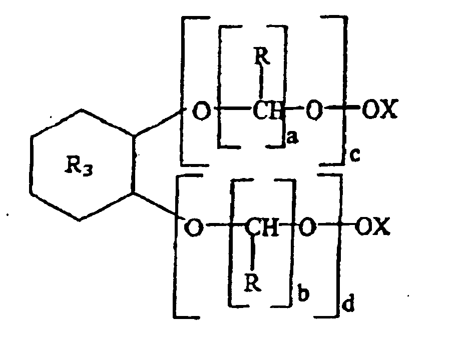 where R₃ - any cyclic hydrocarbon;
R is O, H, OH, CH₃, (CH₂)nCH₃, CH₂OH, (CH₂)nOx or any combinations thereof where n is from 1 to 10.
X is H, sulphate, sulphonate, carboxylate, a pH sensitive group, a mono-, di- or oligiosaccharide or any combination thereof;
a and b are in the range 1-10;
c and d are in the range 1-7; and
the R₃ cyclic hydrocarbon is a C1 to C24 hydrocarbon.

28. A surfactant comprising a compound as claimed in any of Claims 14 to 27.

29. A surfactant as claimed in Claim 28 wherein the surfactant is an anionic surfactant.

30. A surfactant as claimed in Claim 29 wherein the surfactant is a non-ionic surfactant.

31. A surfactant as claimed in Claim 29 further comprising an alkali or alkaline earth metal counter-ion.

32. A method of forming an oil in water microemulsion comprising mixing a solution of a compound as claimed in any of Claims 14 to 27 with an oil for form the oil in water microemulsion.

33. Use of a compound as claimed in any of Claims 14 to 27 in the formation of an oil-in-water microemulsion.

34. A method of forming an oil-in-water microemulsion which comprises mixing a solution of the compound described in Claims 1 to 13 with an oil to form the oil in water microemulsion.

## Patentansprüche

1. Die Verwendung einer Verbindung bei der Zubereitung einer ÖI-in-Wasser-Mikroemulsion, wobei die Verbindung die folgende Allgemeinformel I aufweist: 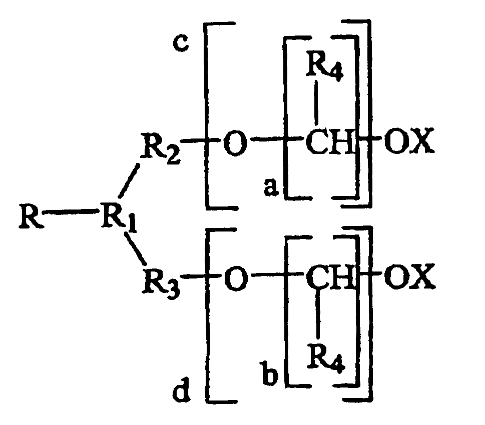 wobei R eine Kohlenwasserstoffgruppe einschließlich H, verzweigten oder linearen Alkylketten, substituiertem Alkyl, Alkenyl, Aryl, substituierten Alkaryl- oder zyklischen Gruppen ist;
R₁ eines aus C, N, P, B, S oder SiO₄ ist;
R₂ eines aus einer kovalenten Bindung, O, CH₂, (CH₂)ₙ ist, wobei n 1-10 Kohlenstoffe lang sein kann;
R₃ eines aus einer kovalenten Bindung, O, CH₂, (CH₂)ₙ ist, wobei n 1-10 Kohlenstoffe lang sein kann;
R₂ und R₃ gleich oder unterschiedlich sein können;
R₄ eines aus O, H, OH, CH₃, (CH₂)ₙCH₃, (CH₂)ₙOH, (CH₂)ₙOX oder jeglichen Kombinationen davon ist, wobei n zwischen 1 und 10 liegt;
OX eine wasserlösliche Gruppe ist;
Ketten a und b jeweils im Bereich von 1-10 Kohlenstoffen liegen; und
die Alkoxyketten c und d jeweils im Bereich von 1-20 Einheiten liegen.

2. Verwendung gemäß Anspruch 1, wobei R ferner eine Ethergruppe oder eine Estergruppe beinhaltet.

3. Verwendung einer Verbindung gemäß Anspruch 1 oder 2, wobei R eine Kettenlänge von C1 bis C30 aufweist.

4. Verwendung gemäß einem der Ansprüche 1 bis 3, wobei R eine Kettenlänge von C3 bis C24 aufweist.

5. Verwendung gemäß einem der Ansprüche 1 bis 4, wobei R eine Kettenlänge von C6 bis C20 aufweist.

6. Verwendung gemäß einem der Ansprüche 1 bis 5, wobei n in der (CH₂)ₙ-Gruppe von R₂ zwischen 1 und 6 liegt.

7. Verwendung gemäß einem der Ansprüche 1 bis 6, wobei n in der (CH₂)ₙ-Gruppe von R₃ zwischen 1 und 6 liegt.

8. Verwendung gemäß einem der Ansprüche 1 bis 7, wobei OX aus der Gruppe ausgewählt wird, die OH, Sulfat, Sulfonat, Carboxylat, Borat oder eine pH-empfindliche Gruppe beinhaltet.

9. Verwendung gemäß Anspruch 8, wobei die pH-empfindliche Gruppe aus der Gruppe ausgewählt wird, die Lacton oder ein Mono-, Di- oder Oligosaccharid beinhaltet.

10. Verwendung gemäß Anspruch 9, wobei das Saccharid Galaktose, Fructose, Saccharose oder Maltose oder eine Kombination davon beinhaltet.

11. Verwendung gemäß einem der Ansprüche 1 bis 10, wobei a und b von 1 bis 8 Kohlenstoffe reichen.

12. Verwendung gemäß einem der Ansprüche 1 bis 11, wobei a und b von 1 bis 4 reichen.

13. Verwendung gemäß einem der Ansprüche 1 bis 12, wobei die Alkoxyketten c und d 2 bis 10 Einheiten beinhalten.

14. Eine Verbindung mit der Allgemeinformel II 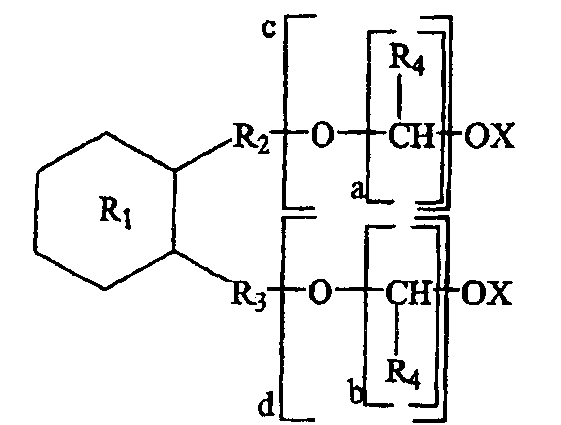 wobei R₁ zyklischer Kohlenwasserstoff ist;
R₂ eines aus einer kovalenten Bindung, O, CH₂, (CH₂)ₙ ist, wobei n 1-10 Kohlenstoffe lang sein kann;
R₃ eines aus einer kovalenten Bindung, O, CH₂, (CH₂)ₙ ist, wobei n 1-10 Kohlenstoffe lang sein kann;
R₂ und R₃ gleich oder unterschiedlich sein können;
R₄ eines aus O, H, OH, CH₃, (CH₂)ₙCH₃, (CH₂)ₙOH, (CH₂)ₙOX ist, wobei n zwischen 1 und 10 liegt;
OX eine wasserlösliche Gruppe ist;
Ketten a und b jeweils im Bereich von 1-10 Kohlenstoffen liegen; und
die Alkoxyketten c und d jeweils im Bereich von 1-20 Einheiten liegen.

15. Verbindung gemäß Anspruch 14, wobei der zyklische Kohlenwasserstoff ein zyklischer C1- bis C24-Kohlenwasserstoff ist.

16. Verbindung gemäß Anspruch 14 oder Anspruch 15, wobei der zyklische Kohlenwasserstoff ein C4- bis C20-Kohlenwasserstoff ist.

17. Verbindung gemäß einem der Ansprüche 14 bis 16, wobei der zyklische Kohlenwasserstoff ein C4- bis C12-Kohlenwasserstoff ist.

18. Verbindung gemäß einem der Ansprüche 14 bis 17, wobei der zyklische Ring mindestens eine Doppelbindung beinhaltet.

19. Verbindung gemäß einem der Ansprüche 14 bis 18, wobei n in der (CH₂)ₙ-Gruppe von R₂ zwischen 1 und 6 liegt.

20. Verbindung gemäß einem der Ansprüche 14 bis 19, wobei OX aus der Gruppe ausgewählt wird, die OH, Sulfat, Sulfonat, Carboxylat, Borat oder eine pH-empfindliche Gruppe beinhaltet.

21. Verbindung gemäß Anspruch 20, wobei die pH-empfindliche Gruppe aus der Gruppe ausgewählt wird, die Lacton oder ein Mono-, Di- oder Oligosaccharid beinhaltet.

22. Verbindung gemäß Anspruch 21, wobei das Saccharid Galaktose, Fructose, Saccharose oder Maltose oder eine Kombination davon beinhaltet.

23. Verbindung gemäß einem der Ansprüche 14 bis 22, wobei a und b von 1 bis 8 Kohlenstoffe reichen.

24. Verbindung gemäß einem der Ansprüche 14 bis 23, wobei a und b von 1 bis 4 reichen.

25. Verbindung gemäß einem der Ansprüche 14 bis 24, wobei die Alkoxyketten c und d 2 bis 10 Einheiten beinhalten.

26. Eine Verbindung mit der Allgemeinformel III 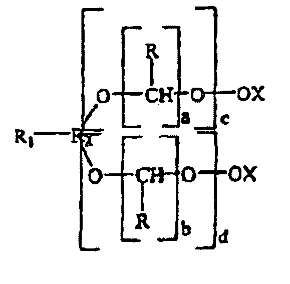 wobei R₁ H, eine verzweigte oder lineare Alkylkette oder ein substituiertes Alkyl, Alkenyl, Aryl, eine substituierte Alkaryl- oder zyklische Gruppe ist;
R₂ eines aus C, N, P, B, S oder SiO₄ oder jeglichen Untergruppen davon ist;
RO, H, OH, CH₃, (CH₂)ₙCH₃, CH₂OH, (CH₂)ₙOX oder jegliche Kombinationen davon ist, wobei n zwischen 1 und 10 liegt;
X H, Sulfat, Sulfonat, Carboxylat, eine pH-empfindliche Gruppe, ein Mono-, Di- oder Oligosaccharid ist;
a und b jeweils im Bereich von 1-10 liegen;
c und d jeweils im Bereich von 1-7 liegen.

27. Eine Verbindung mit der Allgemeinformel IV 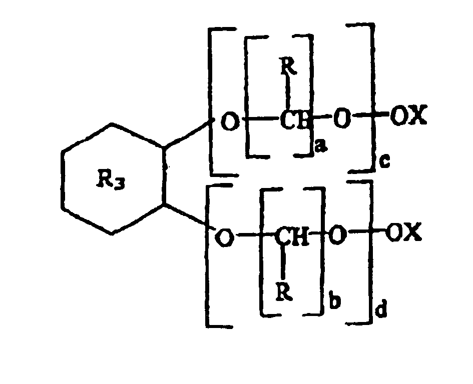 wobei R₃ ein zyklischer Kohlenwasserstoff ist;
RO, H, OH, CH₃, (CH₂)ₙCH₃, CH₂OH, (CH₂)ₙOX oder jegliche Kombinationen davon ist, wobei n zwischen 1 und 10 liegt;
X H, Sulfat, Sulfonat, Carboxylat, eine pH-empfindliche Gruppe, ein Mono-, Di- oder Oligosaccharid oder eine Kombination davon ist;
a und b im Bereich von 1-10 liegen;
c und d im Bereich von 1-7 liegen; und
der zyklische Kohlenwasserstoff R₃ ein C1- bis C24-Kohlenwasserstoff ist.

28. Ein Tensid, das eine Verbindung gemäß einem der Ansprüche 14 bis 27 beinhaltet.

29. Tensid gemäß Anspruch 28, wobei das Tensid ein anionisches Tensid ist.

30. Tensid gemäß Anspruch 29, wobei das Tensid ein nicht-ionisches Tensid ist.

31. Tensid gemäß Anspruch 29, das ferner ein Alkali- oder Erdalkalimetall-Gegenion beinhaltet.

32. Ein Verfahren zum Bilden einer Öl-in-Wasser-Mikroemulsion, das das Mischen einer Lösung einer Verbindung gemäß einem der Ansprüche 14 bis 27 mit einem Öl beinhaltet, um die Öl-in-Wasser-Mikroemulsion zu bilden.

33. Verwendung einer Verbindung gemäß einem der Ansprüche 14 bis 27 bei der Bildung einer ÖI-in-Wasser-Mikroemulsion.

34. Verfahren zum Bilden einer Öl-in-Wasser-Mikroemulsion, das das Mischen einer Lösung der in Ansprüchen 1 bis 13 beschriebenen Verbindung mit einem Öl beinhaltet, um die Öl-in-Wasser-Mikroemulsion zu bilden.

## Revendications

1. Utilisation d'un composé dans la formulation d'une microémulsion huile dans eau, ledit composé ayant la formule générale I 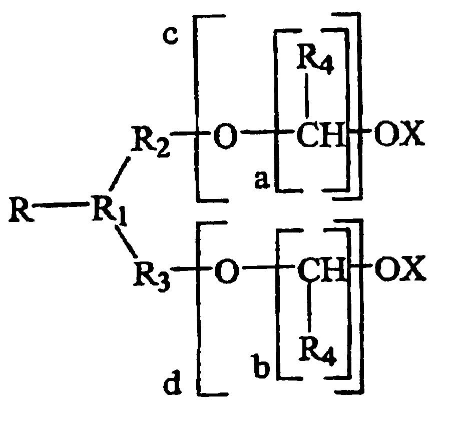 où R est un groupement hydrocarbure comprenant H, des chaînes alkyles ramifiées ou linéaires, alkyle, alcényle, aryle, groupements alkaryles ou cycliques substitués ;
R₁ est n'importe quel élément parmi C, N, P, B, S ou SiO₄;
R₂ est n'importe quel élément parmi une liaison covalente, O, CH₂, (CH₂)ₙ où n peut être de 1 à 10 carbones de long ;
R₃ est n'importe quel élément parmi une liaison covalente, O, CH₂, (CH₂)ₙ où n peut être de 1 à 10 carbones de long;
R₂ et R₃ peuvent être identiques ou différents ;
R₄ est n'importe quel élément parmi O, H, OH, CH₃, (CH₂)ₙCH₃, (CH₂)ₙOH, (CH₂)ₙOX ou toutes combinaisons de ceux-ci où n est compris entre 1 et 10 ;
OX est un groupement soluble dans l'eau ;
les chaînes a et b sont comprises dans la gamme allant de 1 à 10 carbones chacune ; et
les chaînes alkoxy c et d sont comprises dans la gamme allant de 1 à 20 unités chacune.

2. L'utilisation telle que revendiquée dans la revendication 1 dans laquelle R comporte de plus un groupement éther ou un groupement ester.

3. L'utilisation d'un composé telle que revendiquée dans n'importe laquelle de la revendication 1 ou de la revendication 2 dans laquelle R a une longueur de chaîne de C1 à C30.

4. L'utilisation telle que revendiquée dans n'importe laquelle de la revendication 1 à la revendication 3 dans laquelle R a une longueur de chaîne de C3 à C24.

5. L'utilisation telle que revendiquée dans n'importe laquelle des revendications 1 à 4 dans laquelle R a une longueur de chaîne de C6 à C20.

6. L'utilisation telle que revendiquée dans n'importe laquelle des revendications 1 à 5 dans laquelle n dans le groupement (CH₂)ₙ de R₂ est compris entre 1 et 6.

7. L'utilisation telle que revendiquée dans n'importe laquelle des revendications 1 à 6 dans laquelle n dans le groupement (CH₂)ₙ de R₃ est compris entre 1 et 6.

8. L'utilisation telle que revendiquée dans n'importe lesquelles des revendications 1 à 7 dans laquelle OX est sélectionné dans le groupement consistant en OH, sulfate, sulfonate, carboxylate, borate ou un groupement sensible au pH.

9. L'utilisation telle que revendiquée dans la revendication 8 dans laquelle le groupement sensible au pH est sélectionné dans le groupement consistant en lactone, ou en un mono-, di- ou oligo-saccharide.

10. L'utilisation telle que revendiquée dans la revendication 9 dans laquelle le saccharide comporte du galactose, du fructose, du sucrose ou du maltose ou toute combinaison de ceux-ci.

11. L'utilisation telle que revendiquée dans n'importe lesquelles des revendications 1 à 10 dans laquelle a et b sont compris dans la gamme allant de 1 à 8 carbones.

12. L'utilisation telle que revendiquée dans n'importe lesquelles des revendications 1 à 11 dans laquelle a et b sont compris dans la gamme allant de 1 à 4.

13. L'utilisation telle que revendiquée dans n'importe lesquelles des revendications 1 à 12 dans laquelle les chaînes alkoxys c et d comportent de 2 à 10 unités.

14. Un composé ayant la formule générale II 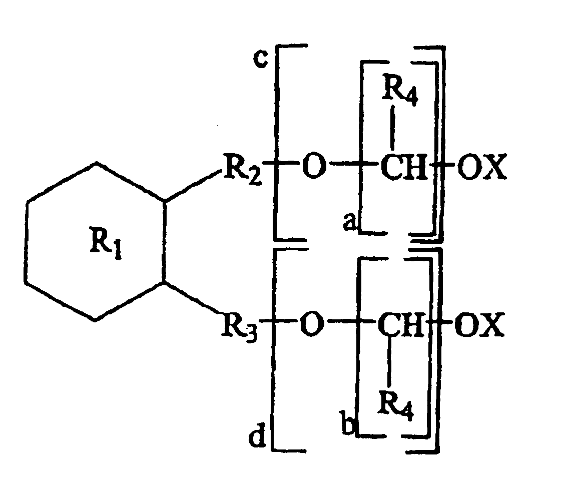 où R₁ est un hydrocarbure cyclique ;
R₂ est n'importe quel élément parmi une liaison covalente, O, CH₂, (CH₂)ₙ où n peut être de 1 à 10 carbones de long ;
R₃ est n'importe quel élément parmi une liaison covalente, O, CH₂, (CH₂)ₙ où n peut être de 1 à 10 carbones de long ;
R₂ et R₃ peuvent être identiques ou différents ;
R₄ est n'importe quel élément parmi O, H, OH, CH₃, (CH₂)ₙCH₃, (CH₂)ₙOH, (CH₂)ₙOX où n est compris entre 1 et 10 ;
OX est un groupement soluble dans l'eau ;
les chaînes a et b sont comprises dans la gamme allant de 1 à 10 carbones chacune ; et
les chaînes alkoxy c et d sont comprises dans la gamme allant de 1 à 20 unités chacune.

15. Un composé tel que revendiqué dans la revendication 14 dans lequel l'hydrocarbure cyclique est un hydrocarbure cyclique C1 à C24.

16. Un composé tel que revendiqué dans la revendication 14 ou la revendication 15 dans lequel l'hydrocarbure cyclique est un hydrocarbure C4 à C20.

17. Un composé tel que revendiqué dans n'importe lesquelles des revendications 14 à 16 dans lequel l'hydrocarbure cyclique est un hydrocarbure C4 à C12.

18. Un composé tel que revendiqué dans n'importe lesquelles des revendications 14 à 17 dans lequel l'anneau cyclique comporte au moins une double liaison.

19. Un composé tel que revendiqué dans n'importe lesquelles des revendications 14 à 18 dans lequel n dans le groupe (CH₂)ₙ de R₂ est compris entre 1 et 6.

20. Un composé tel que revendiqué dans n'importe lesquelles des revendications 14 à 19 dans lequel OX est sélectionné dans le groupement consistant en OH, sulfate, sulfonate, carboxylate, borate ou un groupement sensible au pH.

21. Un composé tel que revendiqué dans la revendication 20 dans lequel le groupement sensible au pH est sélectionné dans le groupement consistant en lactone, ou en un mono-, di- ou oligo-saccharide.

22. Un composé tel que revendiqué dans la revendication 21 dans lequel le saccharide comprend du galactose, du fructose, du sucrose ou du maltose ou toute combinaison de ceux-ci.

23. Un composé tel que revendiqué dans n'importe lesquelles des revendications 14 à 22 dans lequel a et b sont compris dans la gamme allant de 1 à 8 carbones.

24. Un composé tel que revendiqué dans n'importe lesquelles des revendications 14 à 23 dans lequel a et b sont compris dans la gamme allant de 1 à 4.

25. Un composé tel que revendiqué dans n'importe lesquelles des revendications 14 à 24 dans lequel les chaînes alkoxys c et d comportent de 2 à 10 unités.

26. Un composé ayant la formule générale III 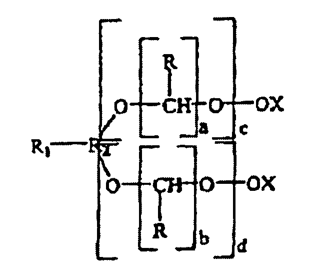 où R₁ est H, une chaîne alkyle ramifiée ou linéaire, ou un alkyle, alcényle, aryle, groupement alkaryle ou cyclique substitué ;
R₂ est n'importe quel élément parmi C, N, P, B, S ou SiO₄ ou n'importe quels sous-groupements de ceux-ci ;
R est O, H, OH, CH₃, (CH₂)ₙCH₃, CH₂OH, (CH₂)ₙOX ou toutes combinaisons de ceux-ci où n est compris entre 1 et 10 ;
X est H, sulfate, sulfonate, carboxylate, un groupement sensible au pH, un mono-, di- ou oligo-saccharide ;
a et b sont compris dans la gamme allant de 1 à 10 chacun ;
c et d sont compris dans la gamme allant de 1 à 7 chacun.

27. Un composé ayant la formule générale IV 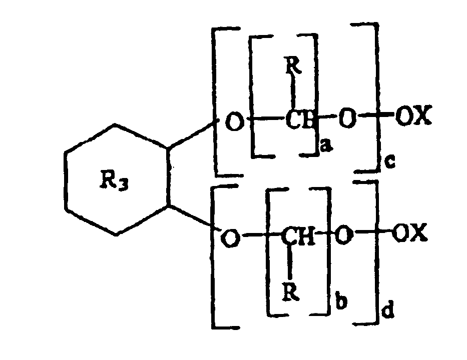 où R₃ - n'importe quel hydrocarbure cyclique ;
R est O, H, OH, CH₃, (CH₂)ₙCH₃, CH₂OH, (CH₂)ₙOX ou toutes combinaisons de ceux-ci où n est compris entre 1 et 10 ;
X est H, sulfate, sulfonate, carboxylate, un groupement sensible au pH, un mono-, di- ou oligo-saccharide ou toute combinaison de ceux-ci ;
a et b sont compris dans la gamme allant de 1 à 10 ;
c et d sont compris dans la gamme allant de 1 à 7 ; et
l'hydrocarbure cyclique R₃ est un hydrocarbure C1 à C24.

28. Un tensioactif comportant un composé tel que revendiqué dans n'importe lesquelles des revendications 14 à 27.

29. Un tensioactif tel que revendiqué dans la revendication 28 dans lequel le tensioactif est un tensioactif anionique.

30. Un tensioactif tel que revendiqué dans la revendication 29 dans lequel le tensioactif est un tensioactif non ionique.

31. Un tensioactif tel que revendiqué dans la revendication 29 comportant de plus un contre-ion métallique alcalinoterreux ou alcalin.

32. Une méthode de formation d'une microémulsion huile dans eau comportant mélanger une solution d'un composé tel que revendiqué dans n'importe lesquelles des revendications 14 à 27 avec une huile pour former la microémulsion huile dans eau.

33. Utilisation d'un composé tel que revendiqué dans n'importe lesquelles des revendications 14 à 27 dans la formation d'une microémulsion huile dans eau.

34. Une méthode de formation d'une microémulsion huile dans eau qui comporte mélanger une solution du composé décrit dans les revendications 1 à 13 avec une huile pour former la microémulsion huile dans eau.
